# EUROPEAN PATENT APPLICATION

(11) **EP 1 875 920 A2**
(43) Date of publication of application: **09.01.2008**
(21) Application number: 07252549.6
(22) Date of filing: 22.06.2007
(51) Int. Cl.: A61K 36/48

(54) **Partially denatured whole soybean extracts and methods of use therefore**

(30) Priority: 23.06.2006 US 473696
(71) Applicant: Johnson and Johnson Consumer Companies, Inc., Skillman, NJ 08558 (US)
(72) Inventor: Seiberg, Miri, Princeton NJ 08540 (US); Stone, Violetta I., Robbinsville NJ 08690 (US); Lin, Connie B., Belle Mead NJ 08502 (US); Zhao, Renbin, Plainsboro NJ 08536 (US); Bruning, Elizabeth, Somerset NKJ 08873 (US)
(74) Representative: Fisher, Adrian John

(57) **Abstract**

This invention relates to compositions containing partially denatured soy products. These compositions can be administered orally to improve aesthetic and general health parameters of an individual.

## Description

### FIELD OF THE INVENTION

This invention relates to compositions containing partially denatured soy products. These compositions can be administered orally to improve aesthetic and general health parameters of an individual.

### BACKGROUND OF THE INVENTION

Aging of the skin is a complex phenomenon resulting from the interaction of several intrinsic and extrinsic factors. Intrinsic aging is an inevitable, genetically programmed process. Among extrinsic influences (e.g., wind, heat, cigarette smoke, chemicals, etc.), ultraviolet radiation appears to be the single most important factor associated with aging of the skin. The effect of ultraviolet radiation on elastic tissues results in elastosis, which is the accumulation of damaged elastin, resulting in reduced elasticity and resilience. (Lam M., Sulindro M., Aging Skin, Academy of Anti-Aging Research MMIII No. 1, pp. 1-8)

Elastin is a critical component of extracellular matrix, and is especially abundant in tissues subject to physical deformations, such as lungs, blood vessels and skin.

The effect of intrinsic aging on tissue elasticity of mucosal tissues (such as vaginal, oral, or rectal mucosal tissues) and of viscero-elastic tissues (that are lining body cavities such as the respiratory track, the gastro-intestinal track, the urinal and bladder track, or the reproductive track), is very similar to the intrinsic aging of the skin. Elastin fiber production in these tissues is reduced with aging, resulting in reduced responsiveness to stimuli. In the oral cavity, such changes can contribute to a decrease in the health of the gums (leading to reduced resistance to the pressure of food processing), increased gum bleeding, loose teeth, and a general decrease in the visual health parameters of the oral cavity (SHIP J., The Influence of Aging on Oral Health and Consequences for Taste and Smell, Physiology & Behavior, Vol. 66, No. 2, pp. 209-215, 1999). In the vagina, reduced elastin fiber production could result in stiffness and reduced sexual function, and uterine prolapse is associated with reduced elasticity of the female reproductive system. Reduced elasticity of the bladder can result in urine incontinence (Castelo-Branco C, Cancelo MJ, Villero J, Nohales F, Julia MD. Management of post-menopausal vaginal atrophy and atrophic vaginitis. Maturitas. 2005 Nov 15;52 Suppl 1:S46-52.). Reduced elasticity of vessel walls can also lead to vessel breakage and bruising. In the eye, degenerative changes in elastin fibers in Brunch's membrane can be responsible for deposition of drusen and macular degeneration (Stone E., et al., Missense Variations in the Fibulin 5 Gene and Age-Related Macular Degeneration. The New England Journal of Medicine 351;346-353). Hemorrhoids, a condition in which the veins around the anus or lower rectum become swollen and inflamed may also be the result of weakening and breakage of collagen and elastin support of the anorectal region. Biochemical studies have identified enhanced levels of elastin-degrading enzymes such as MMP-2 and MMP-9 in hemorrhoidal tissues, which may account for the degraded support network. (Han et al., Pathologic change of elastic fibers with difference of microvessal density and expression of angiogenesis-related proteins in internal hemorrhoid tissues, Zhonghua Wei Chang Wai Ke Za Zhi. 2005;8(1):56-9. Serge D., Etiopathogenesis and physiopathology of hemorrhoidal disease Ann Ital Chir, 1995; 66, (6):747-50. Lierse W, Anatomy and pathophysiology of hemorrhoids Langenbecks Arch Chir Suppl II Verh Dtsch Ges Chir, 1989;:769-72).

Consequently, the reduction in elasticity of these tissues results in reduced quality of life and self esteem. Thus, it is desired to have a treatment that can prevent, retard, or reverse the intrinsic aging effects on tissue elasticity.

Triglycerides are a main constituent of vegetable oil and animal fats, and they play an important role in metabolism as energy sources. However, high triglyceride levels may be associated with a higher risk for atherosclerosis, heart disease, and stroke. (Forrester, J.S., Curr. Opin. Cardiology 2001, 16: 261-264). High triglyceride levels can also increase the risk of thrombosis, which can lead to myocardial infarction (Miller G.J., Atherosclerosis, 2005, 179:213-27).

Hypertriglyceridemia is also a well-known cause of acute pancreatitis, which can have life-threatening complications (Bae J.H. et al., Korean J Gastroenterol. 2005, 46:475-80). Current approaches for lowering triglycerides include diet and pharmacological agents, such as fibric acid derivatives, fish-oil, and CoA reductase inhibitors (Jonkers, I., et al., Am. J. Cardiovasc. Drugs 2001, 1:455-466).

Uric acid is an end product of purine metabolism. Purines are building blocks of RNA and DNA. Most of uric acid produced in the body is excreted by the kidneys. An overproduction of uric acid occurs when there is excessive breakdown of cells, which contain purines, or an inability of the kidneys to excrete uric acid. Hyperuricemia can play a role in the development of gout as well as many degenerative diseases, such as the Metabolic syndrome, which has been linked to a number of coronary heart diseases and increased mortality (Lee, M-Sh., et al., J. Clin. Nutr. 2005, 14:285:292). Hyperuricemia is also involved in the tumor lysis syndrome (TLS), which is a life-threatening constellation of metabolic derangements arising as a consequence of the release of intracellular metabolites by tumor cells as they undergo necrosis (Zeh HJ 3rd, Lotze MT. Addicted to death: invasive cancer and the immune response to unscheduled cell death. J Immunother. 2005;28:1-9). Uric acid and triglycerides were both found to be positively associated with C-reactive protein (CRP) levels (Garcia-Lorda P., et al., C-reactive protein, adiposity and cardiovascular risk factors in a Mediterranean population. International Journal of Obesity (2005) 1-7).

Thus, it is desired to have a treatment that can prevent, retard, or reverse the negative cardiovascular effects induced by high blood levels of triglycerides and uric acid.

Non-denatured whole soybean extracts have been shown to provide anti-aging benefits, including elastin enhancement and reduced uneven pigmentation, when applied topically to the skin, as set forth in copending U.S. Patent Applications Serial Nos. 09/110,409 and 09/698,454, for example, which are hereby incorporated herein by reference. The activity of these extracts is related, in part, to two Soy proteins, Soybean Trypsin Inhibitor (STI), and Bowman Birk Inhibitor (BBI) (see U.S. Patent No. 6,750,229, which is hereby incorporated herein by reference). However, these two proteins alone do not perform as well as the whole soybean extract, suggesting that other, yet unknown components of the soybean contribute to the anti-aging activity of the non-denatured soy extract. Numerous studies have evaluated the effects of technological treatments on the properties of certain soybean protein fractions, in order to identify a fraction with nutritional value but no gastro-intestinal side effects. (Bau HM , Alais C Denaturation and enzymatic proteolysis in vitro of protein fractions of soya flour Ann Nutr Aliment. 1975;29(4):351-70). Technologies such as alkaline soaking and heating to 143°C are commonly used in the nutritional soy industry (Heat treatment of nonfermented oriental soyfoods, p.154-158, in KeShun Liu (ed.) Soybeans, chemistry, technology and utilization, An Aspen publication, Gaithersburg, Maryland 1999). Unfortunately, these technologies result in total protein denaturation. Proteins are "denatured" when their physical and physiological properties are changed, e.g. by heating or change of pH, such that they lose their activity. Such change is generally due to a change in a protein's chemical structure and/or conformation. Protein denaturation and the consequent loss of biological activity are described in biochemistry textbooks (e.g. Biochemistry, A. L. Lehninger, 1975, p.62-63). The soybean-derived protein Bowman-Birk inhibitor (BBI), is a potent chymotrypsin inhibitor that has been extensively studied for its ability to prevent carcinogenesis in many different model systems. (A. Kennedy, Pharmacol Ther. 1998 Jun;78(3):167-209). BBI has been shown to affect skin aging and hair growth when topically applied to the skin, as set forth in U.S. Patent No. 6,750,229. BBI is well tolerated by the gastric system. In clinical trials for head and neck cancers, BBI is used orally, in the form of an ingestible BBI concentrate (BBIC), with no gastric side effects (Kennedy, AR, Overview: anticarcinogenic activity of protease inhibitors. In: W. Troll and AR Kennedy (eds). Protease inhibitors as cancer chemopreventive agents, pp 9-64. New York, Plenum publishing corp, 1993. Kennedy et. A1., Preparation and production of chemopreventive agent, Bowman-Birk inhibitor concentrate, Nutr. Cancer 19: 281- 302, 1993). US Patent Application 2004131711 describes a method of making a BBI concentration including the steps of providing acid extracted solubles from a defatted soybean material; mixing acetone with the acid extracted solubles to form a parts per trillion mixture; separating the part per trillion BBI from the mixture of acetone and acid extracted solubles; diluting the separated part per trillion BBI with water to form an aqueous solution; ultrafiltering the aqueous solution to obtain a retentate.

It is, therefore, an object of this invention, to produce a whole soybean extract with active, non-denatured BBI, but with inactive, denatured STI, to provide a whole soybean ingestible extract substantially devoid of STI, for providing anti-aging benefits in an ingestible form.

### SUMMARY OF THE INVENTION

This invention relates to the unexpected discovery that partially denatured whole soybean extract is effective, upon ingestion, for enhancing the elasticity or structural integrity of the skin, vaginal, urogenital, and mucosal tissues as well as for reducing triglyceride and uric acid levels in the blood and urine.

Accordingly, this invention relates to compositions containing partially denatured whole soybean extract of having active Bowman-Birk Inhibitor (BBI) but are substantially devoid of or having reduced trypsin inhibitory activity (e.g. STI) that is not tolerable by the digestive system and a method of making such compositions. In accordance with the methods of this invention, soy trypsin inhibitor is selectively inactivated by a method of processing the beans utilizing differential heating.

Thus, in one aspect, this invention relates to a composition containing a partially denatured whole soybean extract having active Bowman-Birk Inhibitor (BBI) and having reduced or eliminated trypsin inhibitory activity. Furthermore, the compositions of this invention may also contain one or more acceptable carriers for ingestible formulation.

In another aspect, this invention relates to a method for enhancing aesthetic and general health parameters of an individual by orally administering to the individual in need of a composition that contains partially denatured whole soybean extract containing active Bowman-Birk Inhibitor (BBI) but having reduced trypsin inhibitory activity. Said method could reduce or help in delaying or preventing the following conditions: aging symptoms in skin, could improve urinary incontinence, could prevent or reduce the severity of hemorrhoidal conditions, and could lower cardiovascular disease risk factors, by enhancing the elasticity or the structural integrity of the skin, vaginal, urogenital, and mucosal tissues as well as reducing triglyceride and uric acid levels in the blood and urine.

### DETAILED DESCRIPTION OF THE INVENTION

We believe that one skilled in the art can, based upon the description herein, use the present invention to its fullest extent. The following specific embodiments are to be construed as merely illustrative, and do not serve to limit the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference. Unless otherwise indicated, a percentage refers to a percentage by weight (i.e., %(W/W)).

### Definitions

"Enhancing the elasticity or the structural integrity" means increasing or improving the synthesis of elastic fibers, preventing the loss, or retarding the loss of elasticity or structural integrity of the tissue, including but not limited to, treating sagging, lax and loose tissue, tightening skin or mucosal or viscero-elastic tissues.

The loss of elasticity or tissue structure integrity may result from a number of factors, including but not limited to: disease, aging, hormonal changes, mechanical trauma, environmental damage, or the result of an application of products, such as a cosmetics or pharmaceuticals, to the tissue.

"Reducing triglyceride or uric acid" means, respectively, reducing the level, or preventing the increase in the level, of triglycerides in serum or of uric acid in the serum and/or urine.

The increase in triglyceride or uric acid levels in the body may be a result of a number of factors, including but not limited to disease, aging, hormonal changes, environmental damage, genetic factors, nutritional imbalance, and the like.

"Mucosal tissues" are tissues that express elastin and are composed in part of cells of mesenchymal and epithelial origin. Examples of mucosal tissues include, but are not limited to, vaginal, oral, corneal, nasal, rectal, and viscero-elastic tissues. Examples of viscero-elastic tissues are those that line the respiratory track, blood vessel walls, the gastro-intestinal track, the urinal track, the bladder, and the reproductive track.

"Urogenital tissues" are tissues of the bladder, the urinal track and the reproductive track, including but not limited to the vagina, clitoris, external genitalia, uterus, bladder, and urethra.

"Vessel walls" are walls of vessels of the circulatory system that function to transport blood or lymph throughout the body. The most important types of blood vessels, arteries and veins, carry blood away from or towards the heart, respectively.

A "product" is a product in finished packaged form. In one embodiment of the products of this invention, the package is a container such as a paper, cardboard, plastic, metal or glass bottle or jar containing the composition or a "blister pack" for containing unit dosages of the composition. The product may further contain additional packaging such as a plastic or cardboard box for storing such container. In one embodiment, the product contains instructions directing the user to ingest the composition (e.g., for the purposes set forth herein). Such instructions may be printed on the container, label insert or on any additional packaging.

Various methods of using the compositions of this invention and statements that may be used to "promote" the sale of such compositions are listed below. What is meant by "promoting" is promoting, advertising, or marketing and such promoting relates to various methods of using the compositions of the invention in accordance with this invention. Examples of promoting include, but are not limited to, written, visual, or-verbal statements made on the product or in stores, magazines, newspaper, radio, television, internet, and the like. Examples of such statements include, but are not limited to, "enhances skin elasticity or structural integrity," "improving visible and tactilely perceptible manifestations of the skin," "increases skin elasticity or structure," "restores skin elasticity or structure," "treats sagging of lax skin," "enhances vaginal elasticity," "enhances sexual satisfaction," "increases vaginal elasticity," "restores vaginal elasticity," "strengthen vaginal wall," "prevents or treats vaginal prolapse," "reduces incontinence episodes," "strengthen bladder wall," "improves bladder compliance," "enhances gum elasticity," "increases gum elasticity," "restores gum elasticity," "enhances alveolar wall elasticity," "increases alveolar wall elasticity," "enhances the healthy look of the gums," "restores alveolar wall elasticity," , "decreases the risk of hemorrhoids", "Prevents or reduces pain and discomfort from hemorrhoids", " reduces the severity of hemorrhoids", "decreases the risk of cardiovascular diseases," "decreases the risk of pancreatitis," "reduces pancreatitis," "decreases the risk of thrombosis," "reduces thrombosis," "lowers C-reactive protein (CRP)," "decreases systolic and diastolic blood pressure," "lowers tissue edema," "reduces gout," "reduces tumor lysis syndrome," "slows or reverses age-related metabolic syndromes," and "slows or reverses disease-related metabolic syndromes." "enhances vessel wall strength," "improves fragile skin," "decreases bruising," and "decreases inflammatory cell extravasation."

As used herein, "ingestible composition" means a composition that is intended to be ingested. Examples of forms of ingestible compositions include, but are not limited to, tablets, pills, capsules, powders, granules, solutions or suspensions, and drops. Such compositions may be swallowed whole or may be in chewable form. An "ingestible composition" may also be in the form of a confectionary or a food product such as a cookie, candy, food bar, chewing gum, yogurt additive, sprinkles, tea, juice or other drink, liquid shake or the like. Ingestible compositions do not include compositions intended to be topically administered to the skin or oral/vaginal cavity.

As used herein, "pharmaceutically-acceptable" means that the ingredients which the term describes are suitable for ingesting without undue toxicity, incompatibility, instability, irritation, allergic response, and the like.

As used herein, "safe and effective amount" means an amount of the extract or of the composition sufficient to induce the desired effect, but low enough to avoid serious side effects. The safe and effective amount of the compound, extract, or composition will vary with e.g. the age, health and environmental exposure of the end user, the duration and nature of the treatment, the specific extract, ingredient, or composition employed, the particular pharmaceutically-acceptable carrier utilized, and like factors.

### Soy extract:

In one embodiment, partially-denatured whole soybean extract of the compositions of this invention is a substance derived from the soybean, containing ingredients naturally found in soybeans, at the relative concentrations as found in the beans, with the exception of water, in which said extract contains active BBI but reduced or eliminated trypsin inhibitory activity. The partially denatured soy extract of this invention may be in the form of a fluid (e.g., soymilk) or a solid (e.g., a soybean powder or soymilk powder). When in the form of a fluid, the term "soy" refers to the solid constituents of the fluid that are derived from the soybean. The soy may be in the form of soybean powder. Soybean powder may be made by grinding dry soybeans. The soybean powder may be lyophilized. Soymilk and soymilk powder are also useful soy products. Soymilk is a combination of solids derived from soybeans and water, the mixture of which has some or all of the insoluble constituents filtered off.

Soymilk powder is evaporated soymilk, which in one embodiment, is in a lyophilized, or freeze-dried or spray-dried form. Procedures for manufacturing soymilk include, but are not limited to, the following three: (a) soymilk may be made by placing soybeans into water to allow them to absorb the water. The swelled beans are then ground and additional water is then added. The mixture may then be filtered to remove any insoluble residue. A method according to this invention, utilizing this procedure, is described more fully below. Soymilk may also be prepared from soybean powder. Soybean powder is thoroughly mixed with water (e.g., depending upon the efficiency of the mixing process, for at least one hour), and then followed by filtering the resulting mixture to remove insoluble residues. (c) soymilk may also be reconstituted from soymilk powder by adding water. The soymilk useful in the compositions of this invention may comprise from about 1% to about 50%, by weight (more preferably, from about 5% to about 20%, by weight) of solids from the soybean.
Another example is the use of soymilk powder, made from lyophilized, spray dried or freeze-dried soymilk, with the addition of water and following with or without additional filtration or homogenization. Other methods of soybean extraction could also be used to create the active ingredients in the formulations described below. For example, the active ingredients could be extracted from ground soybeans using ethanol/water mixtures, followed by the removal of the ethanol from the extract, in such ways that the BBI activity of the soybean will be retained, but the STI activity will be removed or substantially reduced.
The soymilk useful in the compositions of this invention may comprise from about 1% to about 50%, by weight (more preferably, from about 5% to about 20%, by weight) of solids from the soybean. The abovementioned processes yield a substrate which may be processed, in accordance with the methods of this invention, to yield the novel compositions of this invention having reduced trypsin inhibition while retaining BBI activity.

Other known active ingredients of soy include, but are not limited to, isoflavones, phytoestrogens, genistein, daidzein, glycitein, saponins, and phytosterols. The soy products useful in the compositions of this invention may be produced from all soybean species, regardless of their geographic origin, genetic origin, sun exposure, harvest time and the like. However, specific strains, geographic origins or growth conditions might be preferred. For example, preferred soybean strains are those particularly rich in their BBI content, or particularly low in their STI content. Growth conditions resulting in BBI enrichment or in STI reduction in the bean are also preferred.

"Denaturation" is defined as the change in the physical and the physiological properties of a protein, that is brought about by heat, X-rays, organic solvents or other chemicals. These changes include loss of activity (e.g. for enzymes) and loss (or alteration) of antigenicity (in the case of antigens).

A "partially denatured whole soybean extract" is a composition extracted or derived from the whole soybean in which the processing for the derivation of such soy extract (e.g., the temperature, duration at certain temperature, extraction media) selectively and substantially inactivates the trypsin inhibitory activity of soybean trypsin inhibitor (STI) protein, while preserving the activity of the Bowman-Birk inhibitor (BBI).

Preferably, the soy products utilized in the compositions and methods of this invention should have a microbial content of less than about 1,000 cfu per gram (such as less than about 100 cfu per gram) of the legume product. In order to achieve such low microbial content, the soy products of this invention may be exposed to gamma irradiation in accordance with the procedures set forth in U.S. Patent Nos. 6,555,143 (Issued April 29, 2003) and U.S. Patent Application Serial No. 09/796,054 filed February 28, 2001, which are both incorporated herein by reference. The soy product may be exposed to between about 2 to about 30 kGy of gamma irradiation, such as between about 5 and about 10 kGy of gamma irradiation. Surprisingly, such treatment reduces the microbial content of the legume product of this invention, while maintaining its biological activity (e.g. retaining BBI activity). The treatment of legume products with gamma irradiation maintains the cosmetic elegance of the legume product, such as maintained natural colors and does not induce significant malodors.

Other anti-microbial processes that also maintain the protease inhibitory activity of the legume product that can be practiced alone or in combination with gamma irradiation, include, but are not limited to, exposure to x-rays, high energy electron or proton beams, ultraviolet radiation, hydrostatic pressure, and addition of chemical agents possessing antimicrobial activity that do not affect the biological activity profile of the compositions of this invention, and combinations thereof.
The partially denatured whole soybean extract of this invention may be combined with continues coggygria extract and/or with malva sylvestris extract in order to achieve additional efficacy in promoting elasticity in the tissues of the body. Compositions containing coggygria extract and malva sylvestris extract are described in copending patent applications U.S. Serial Nos. 10/973,313 filed October 26, 2004, 11/313/079 filed December 20, 2005, 11/248,465 filed October 12, 2005 and 11/387,892 filed March 23, 2006, which are hereby incorporated herein by reference.

Preferably, partially denatured whole soybean extract is present in the composition in an amount from about 0.001% to about 20% by weight, in particular in an amount from about 0.01% to about 10% by weight. Unless stated otherwise, the weight of the extract refers to the dry weight of the extract.

### Ingestible Compositions

The ingestible compositions useful in the present invention involve formulations suitable for ingesting by the mammal, such as a human, in need to such treatment. In one embodiment, the compositions contain a safe and effective amount of (i) partially denatured whole soybean extract and (ii) a pharmaceutically-acceptable carrier.

In one embodiment, the extracts of this invention are combined with foods, confectionary or food supplements; said foods, confectionary or food supplements were not processed to temperature higher than 90°C after the addition of extracts of this invention, and said food supplements having a nutritional or physiological effect whose purpose is to supplement the normal diet. Said nutritional supplements are in the form of botanical extracts, synthetic molecules and mixtures thereof. Said nutritional supplements include, but are not limited to proteins, vitamins (such as vitamin A or its derivatives or different forms of tocopherols), Minerals (such as Zinc, Iron or Selenium), antioxidants (such as ascorbate, lycopenes or carotenes), Fatty acids (such as omega-3 fatty acids). Other examples of nutritional supplements that can we combined with extracts of this invention include, but are not limited to Soy extracts, pomegranate juice, green tea and phenolic compounds and synthetic derivatives of resveratrol and the like. Said foods and confectionary include, but are not limited to candy, chocolate, chewing gum, bars, snack foods, dairy products such as yogurt, pastry and baked goods and the like. However, these products should not be heated to a temperature higher than 90°C after the addition of extracts of this invention.

In one embodiment, the ingestible compositions herein contain, per dosage unit, e.g., tablet, capsule, powder, injection, teaspoonful and the like, or a food or a confectionary dose unit, an amount of the extract(s) necessary to deliver an effective dose as described above. Preferably, the ingestible compositions herein contains, per dosage unit, e.g., tablet, chewable tablet, capsule, powder, teaspoonful and the like, of from about 5 to about 2500 mg, such as from about 1 to about 2000 mg, and may be given at a dosage of from about 10 mg/kg/day to about 5g/kg/day, such as from about 0.1 gr/kg/day to about 1 gr/kg/day. More preferably, a dosage of about 500 to about 1000 mg/kg/day should be used. In another embodiment the ingestible compositions herein may be incorporated into foods and confectionary products, from about 5 to about 5000 mg per food or confectionary product unit.

The preferred dosages, however, may be varied depending upon the requirement of the individuals, the severity of the condition being treated, and the extract(s) being employed. The use of either daily administration or post-periodic dosing may be employed. In one embodiment, the compositions of this invention may be provided in the form of tablets, such as those containing 50, 100, 250, 500, 1000, 2000 and/or 5000 milligrams of the extract(s) for the symptomatic adjustment of the dosage to the individual to be treated. The extract(s) may be administered on a regimen of 1 to 4 times per day. Advantageously, the compositions may be administered in a single daily dose, or the total daily dosage may be administered in divided doses of two, three or four times daily.

Optimal dosages to be administered may be readily determined by those skilled in the art, and will vary with the particular extract(s) used, the mode of administration, the strength of the preparation, and the advancement of the disease/ condition being treated. In addition, factors associated with the particular individual being treated, including individual's age, weight, diet and time of administration, will result in the need to adjust dosages.

Ingestible compositions containing one or more of the extracts of the invention described herein can be prepared by intimately mixing the extract(s) with a pharmaceutically-acceptable carrier according to conventional pharmaceutical compounding techniques, providing that the extracts of this invention are not exposed to a temperature higher than 90°C. The carrier may take a wide variety of forms depending upon the type of formulation. Thus for liquid preparations such as suspensions, elixirs and solutions, suitable carriers and additives include water, glycols, oils alcohols, flavoring agents, preservatives, stabilizers, coloring agents and the like; and for solid preparations, such as powders, capsules and tablets, suitable carriers and additives include starches, sugars, diluents, granulating agents, lubricants, binders, disintegrating agents and the like. Solid oral preparations may also be coated with substances such as sugars or be enteric-coated so as to modulate major site of absorption.

For preparing solid compositions such as tablets, the principal extract(s) is mixed with a pharmaceutically-acceptable carrier, e.g. conventional tableting ingredients such as corn starch, lactose, sucrose, sorbitol, talc, stearic acid, magnesium stearate, dicalcium phosphate or gums, and other pharmaceutically-acceptable diluents, e.g. water, to form a solid preformulation composition containing a homogeneous mixture of the extract(s). When referring to these preformulation compositions as homogeneous, it is meant that the extract(s) is dispersed evenly throughout the composition so that the composition may be readily subdivided into equally effective dosage forms such as tablets, pills and capsules. This solid preformulation composition may then subdivided into unit dosage forms of the type described above. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

The liquid forms in which the novel compositions of the present invention include aqueous and/or organic (e.g., ethanol) solutions, suitably flavored syrups, aqueous or suspensions, and flavored emulsions with edible oils such as cottonseed oil, sesame oil, coconut oil or peanut oil, as well as elixirs and similar pharmaceutically-acceptable vehicles, when such vehicle have no effect on BBI activity. Suitable dispersing or suspending agents for aqueous suspensions, include synthetic and natural gums such as tragacanth, acacia, alginate, dextran, sodium carboxymethylcellulose, methylcellulose, polyvinyl-pyrolidone or gelatin.

One skilled in the art will recognize that, both in vivo and in vitro trials using suitable, known and generally accepted cell and/or animal models are predictive of the ability of an extract to treat or prevent a given condition. One skilled in the art will further recognize that human clinical trails including first-inhuman, dose ranging and efficacy trials, in healthy individuals and/or those suffering from a given condition or disorder, may be completed according to methods well known in the clinical and medical arts.

### Example 1: Soy Extract Preparations

The following is a description of the preparation of various extracts of the present invention. As used in the subsequent Examples, the weight percentage of extract refers to the weight of the soybean powder.

Extract 1A: Devansoy "high sucrose" non-denatured soybean powder was purchased from Dupont, Wilmington DE. 2% non-denatured soybean extract suspension was made in PBS, with brief sonication of 3 repeats of 45 seconds on ice, and heat-denatured for 1 hour at 90°C (Extract 1A). Extract 1B: Soy preparation was also made by suspending non-denatured soybean powder (conventional non-GMO soybeans, Natural Products, Inc., Grinnell, IA) in 1x PBS at a concentration of 2% (w/v). The suspension was sonicated 3-4 times for 10 seconds at 70% power (using Sonics CV33, Newtown, CT) to make a homogeneous suspension and heated at different temperatures and durations (Extract 1B). The homogeneous suspension of extracts 1B was then diluted to 0.2% soy (w/v) as a working solution. STI and BBI (Sigma, St Louis, MO) were dissolved in 1x PBS at a concentration of 0.05% (w/v), respectively. Preparations of Soy (0.2%, w/v), STI (0.05%, w/v), and BBI (0.05%, w/v) were then incubated at different temperature (0-100°C) for 1 hour. The heat-treated samples were later tested for STI and BBI activity using trypsin and chymotrypsin inhibition assays, respectively.

The inhibition of trypsin (representing STI and BBI activity) or chymotrypsin (representing BBI activity)-induced cleavage of a fluorescent casein peptide was measured using the EnzChek^{™} protease assay kit, following manufacturer's instructions (EnzChek^{™} Protease Assay Kits Product Information, Revised 3/15/99; Molecular Probes, Eugene OR). Briefly, soy preparations (Extract 1B, 0.2%, w/v), or STI, or BBI solutions (0.05%) were incubated with 100 units of trypsin or 0.08 units of chymotrypsin (Sigma, St. Louis, MO) dissolved in digestion buffer provided in the assay kit. Then, 1.0 mg/ml stock solution of BODIPY FL casein was prepared by adding 0.2 mL of deionized water to the vials supplied with this substrate (provided in kit), then made to a final working concentration of 10 microgram/ml in digestion buffer. Following incubation of the trypsin or chymotrypsin, with or without the test material, with the BODIPY fluorescent casein substrate, at room temperature for one hour, fluorescence was measured (excitation 485 nm /emission 530 nm) using a SpectraMax Gemini microtiter plate reader (Molecular Devices Corporation, Sunnyvale, CA) and Softmax Pro 3.0 software (Molecular Devices Corporation). Each experiment was performed in three replicates. The results of the experiment are shown in Table I. These results demonstrate that it is possible to inhibit STI (trypsin inhibitory activity) without inhibiting BBI. These data suggest that heating non-denatured soy extracts to e.g. 90°C for 1 hr, would reduce or eliminate trypsin inhibitory activity of STI but preserve the BBI activity of the soy preparation.

**Table I. Trypsin and Chymotrypsin Inhibition Activity**

| Temperature (°C) | % trypsin inhibition Activity | | | % Chymotrypsin inhibition activity | |
|---|---|---|---|---|---|
| | 0.05% BBI | 0.05% STI | 0.2% Soy | 0.05% BBI | 0.2% Soy |
| 0 | 93.97 | 82.47 | 33.51 | 92.53 | 66.76 |
| 4 | 93.92 | 82.78 | 34.43 | 92.76 | 67.16 |
| RT | 94.17 | 82.78 | 33.76 | 92.6 | 66.57 |
| 40 | 94.02 | 83.19 | 33.76 | 92.6 | 66.18 |
| 50 | 93.56 | 81.6 | 37.5 | 92.5 | 66.31 |
| 60 | 94.43 | 79.2 | 37.04 | 94.23 | 64.68 |
| 70 | 95.14 | 81.35 | 36.06 | 96.05 | 62.98 |
| 80 | 97.39 | 75.78 | 34.28 | 97.98 | 62.26 |
| 90 | 97.24 | 74.4 | 31.21 | 97.95 | 62.43 |
| 100 | 28.71 | -1.81 | 5.86 | 66.67 | 19.28 |

### Example 2: Enhancement of elastic fiber network in mouse bladder and skin by oral treatment with a partially denatured whole soybean extract.

C57BL/6 female mice of age 5 weeks were purchased from Taconic Farms (Germantown, NY). Mice were housed in appropriately sized cages in an environmentally controlled room with a 12-hour light-12-hour dark photoperiod and supplied with food and water *ad libitum.* Animal care was based on the "Guide for the Care and Use of Laboratory Animals", NIH Publication No. 85-23. Animals were acclimated for 3 weeks before study starts, using a special Casein Based Diet (5K96 with low isoflavone content, purchased from Test Diet) and housed together to achieve synchronized estrous cycling. After acclimation, 200µl of test materials (Extract 1A) were given orally, 5 days a week Monday through Friday. Skin and bladder samples following 12 and 22 weeks of treatment were obtained for histological analysis. Oral treatment continued for 22 weeks. During this period the animals appeared healthy, did not lose body weight, and did not show any signs of digestive distress.

The effect of selected partially denatured whole soybean extract on mouse bladder and skin was assessed on C57Bl/6 mice. 12 weeks after the start of the oral treatments, mice were sacrificed, and bladder and skin samples were analyzed histologically for elastin fibers. Surprisingly, an increased amount of elastic fibers was observed in mouse bladders and skins from all mice treated orally with the partially denatured soybean extract, as compared to controls.

**Table II. Elastin fiber density - week 12**

| Group | Bladder | Skin |
|---|---|---|
| Control | + | + |
| Partially-denatured Soy extract 1A, 5% | +++ | ++ |
| Grading | | |
| normal elastin level (control): + | | |
| slightly increased: ++ | | |
| moderately increased: +++ | | |

The ingestible treatments were continued for additional two months, and bladder and skin samples at week 22 after the start of the treatments were analyzed histologically for elastin fibers. Surprisingly, an even greater increase in elastic fibers was observed in mouse bladders and skins of mice treated with the partially denatured soybean extract, as compared to control and to results from week 12.
Elastin fiber density around bladder vessels was also evaluated. As shown in Table III, there was a considerable increase in the elastin fiber density around blood vessels in bladders of treated mice, as compared to controls. Results of 22-week treatment are shown in Table III.

**Table III - Elastin fiber density - week 22**

| | | Bladder | | Bladder vessels | Skin |
|---|---|---|---|---|---|
| Control | | + | | + | + |
| Partially-denatured Soy | | +++ | | | |
| extract 1A, 5% | | (1/3 of mice) | | +++ | +++ |
| | Grading | | | | |
| | normal elastin level (control): + | | | | |
| | slightly increased: ++ | | | | |
| | moderately increased: +++ | | | | |

This example demonstrates that the use of partially denatured whole soybean extracts is well tolerated, causes no digestive distress, and provides elastin and structural enhancement to body tissues.

### Example 3: Improvement in systemic health parameters by oral treatment with a selected partially denatured whole soybean extract.

Mice were treated as in Example 2, and the effect of selected partially denatured whole soybean extract on mouse plasma triglyceride and uric acid levels was assessed. Three months after the start of the oral treatments, mice were sedated by isoflurane gas inhalation, and blood was collected by heart puncture. Surprisingly, a major decrease in the levels of triglycerides and uric acid was observed in plasma from mice treated orally with the selected natural extracts, as compared to controls.

**Table IV. Change in blood parameters**

| Groups | Triglycerides mg/(ml) dl | Uric acid mg/dl |
|---|---|---|
| Control | 87 +/- 3 | 3.75 +/- 0.25 |
| partially denatured whole soybean extract 1A, 5% | 54.5 +/- 6.5 | 2.8 +/- 0.1 |

## Claims

1. An ingestible composition, said composition comprising a partially denatured whole soybean extract.

2. A composition according to claim 1 wherein said composition comprises soybean-derived Bowman-Birk inhibitory activity and has substantially reduced soybean-derived trypsin inhibitory activity in comparison with nondenatured whole soybeans.

3. A composition according to claim 1, wherein said composition further comprises one or more pharmaceutically-acceptable carrier.

4. A composition according to claim 2 wherein said composition comprises chymotrypsin inhibitory activity, which inhibits at least about 0.08 units of chymotrypsin to about 50% or more and trypsin inhibitory activity, which inhibits 100 units of trypsin to about 40% or less when the composition contains 0.2% soy extract.

5. A composition according to claim 1 wherein said partially denatured whole soybean extract is selected from the group consisting of: soymilk, soybean powder and soymilk powder.

6. A composition according to claim 5 wherein said partially denatured whole soybean extract is soymilk powder.

7. A composition according to claim 4 wherein said composition may be safely ingested.

8. A composition according to claim 2 wherein said composition further comprises nutritional supplements consisting of vitamins, minerals, proteins, peptides, fatty acids, antioxidants, botanical extracts and mixtures thereof.

9. An ingestible composition comprising partially denatured whole soybean extract and one or more pharmaceutically-acceptable carrier(s) comprising from about 5 to about 5000 milligrams of partially denatured whole soybean extract.

10. An ingestible composition according to claim 9 wherein said composition comprises from about 50 to about 2000 milligrams of partially denatured whole soybean extract.

11. A nutritional supplement composition comprising partially denatured whole soybean extract and selected from the group consisting of a drink, a chewable tablet, a shake, a food and a confectionary.

12. A nutritional supplement according to claim 11 wherein said food is selected from the group consisting of a candy, chocolate, chewing gum, a bar, a snack food, confectionary, sprinkles, a dairy product, a pastry and a cookie.

13. A method of preparing a partially denatured whole soybean extract comprising
a) providing a whole non-denatured soy material in an aqueous carrier;
b) heating said soy material for a period of time and at a temperature sufficient to reduce or substantially eliminate trypsin inhibitory activity in said extract and insufficient to denature or to inactivate BBI in said extract.

14. A method according to claim 13 wherein said period of time is from about 40 to about 100 minutes.

15. A method according to claim 13 wherein said temperature is from about 80°C to about 95°C.

16. A method according to claim 13 wherein said period of time is from about 40 to about 100 minutes and said temperature is from about 80°C to about 95°C.

17. A method of claim 16, wherein said temperature is about 90°C.

18. A method of claim 13 wherein said solution is heated at about 90°C for about 60 minutes.

19. A method of increasing elastin fiber network of a mammal comprising said mammal's ingesting a composition comprising a partially denatured whole soybean extract.

20. A method of decreasing triglycerides in the serum of a mammal comprising said mammal's ingesting a composition comprising a partially denatured whole soybean extract.

21. A method of decreasing uric acid in the serum or urine of a mammal comprising said mammal's ingesting a composition comprising a partially denatured whole soybean extract.

22. A method of enhancing the elasticity or structural integrity of skin, urogenital tissue, anorectal tissue, vessels or mucosal tissue of a mammal in need of such enhancement, said method comprising the ingestion by said mammal of a composition consisting of a partially denatured whole soybean extract.

23. A method according to claim 22, wherein said method comprises enhancing the elasticity or structural integrity of the skin.

24. A method according to claim 22, wherein said method comprises enhancing the elasticity or structural integrity and function of mucosal tissue.

25. A method according to claim 22, wherein said method comprises enhancing the elasticity or structural integrity of urogenital tissue.

26. A method according to claim 22, wherein said method comprises enhancing the elasticity or structural integrity of bladder tissue.

27. A method according to claim 22, wherein said method comprises enhancing the elasticity or structural integrity of anorectal tissue.

28. A method according to claim 22, wherein said method comprises enhancing the elasticity or structural support and function of a vessel wall.

29. A method according to claim 22, wherein said method comprises enhancing the elasticity or structural integrity and function of vaginal tissue.
